Europäisches Patentamt

European Patent Office

Office européen des brevets

(11) Publication number: **0 048 194**
A2

(12) **EUROPEAN PATENT APPLICATION**

(21) Application number: **81401347.0**

(22) Date of filing: **27.08.81**

(51) Int. Cl.³: **F 26 B 5/06**
**A 01 N 1/02**

(30) Priority: **28.08.80 US 182242**

(43) Date of publication of application:
**24.03.82 Bulletin 82/12**

(84) Designated Contracting States:
**AT BE CH DE FR GB IT LI LU NL SE**

(71) Applicant: **MERCK & CO. INC.**
**126, East Lincoln Avenue P.O. Box 2000**
**Rahway, New Jersey 07065(US)**

(72) Inventor: **Hilleman, Maurice R.**
**4107 Fields Drive**
**Lafayette Hill Pennsylvania 19444(US)**

(72) Inventor: **Hurni, William M.**
**329 Evergreen Drive**
**North Wales Pennsylvania 19454(US)**

(74) Representative: **Corre, Jacques Denis Paul et al,**
**Cabinet Regimbeau 26, Avenue Kléber**
**F-75116 Paris(FR)**

(54) **Lyophilization process.**

(57) Lyophilization time and expense are reduced by shell freezing the liquid vaccine prior to lyophilization by rotating the vial on its side in a liquid bath maintained at a temperature below the eutectic point of the vaccine.

Fig.1.

EP 0 048 194 A2

-1-

LYOPHILIZATION PROCESS

BACKGROUND OF THE INVENTION

Aqueous solutions of materials which are known to be unstable, e.g., vaccines, are stabilized by removal of water by lyophilization. Lyophilization, however, is a time-consuming and energy intensive process, and requires the use of expensive equipment.

At present most industrial techniques for freezing pharmaceuticals prior to lyophilization involve freezing the pharmaceutical at the bottom of a vial (plug freezing), or freezing two or more separated strata (stratified plug freezing), or slant freezing.

Plug freezing is most common, being the simplest and cheapest. In this method the vials are placed vertically on trays which are then transferred to refrigerated shelves or immersed in freezing baths. As the freezing proceeds from the outer to the inner part of the vial, an outer layer of frozen liquid is set up which impedes efficient heat transfer; as a consequence, long treatment at low temperature is required to freeze the liquid completely. Stratified plug freezing, employed to avoid the mixing of inter-reacting substances, suffers from the same disadvantages.

-2-

Slant freezing or double slant freezing, by tilting the vial once or twice at different angles, produces a frozen liquid with varying thickness. This fails to overcome the foregoing disadvantages as lyophilization time is a function of the greatest thickness of the frozen liquid.

Rotating paraboloid freezing produces frozen material whose inner surface acquires a parabolic shape. This technique also produces frozen material having varying thickness and has the accompanying disadvantages.

OBJECTS OF THE INVENTION

It is an object of the present invention to provide an improved lyophilization process. Another object is to provide a faster and more economical lyophilization process. Another object is to provide a high speed, continuous flow, shell freezing system which is compatible with a modern high speed filling line. These and other objects of the present invention will be apparent from the following description.

SUMMARY OF THE INVENTION

Lyophilization time is reduced and costs per unit of lyophilized product are reduced by shell freezing the material to be lyophilized in the lyophilization vessel prior to lyophilization. The shell freezing is effected by rotating the vial on its side in a liquid bath having a temperature below the eutectic point of the vaccine.

BRIEF DESCRIPTION OF THE DRAWING

Figure 1 is a perspective view of a lyophilization vial, and

Figure 2 is a plan view taken along the line 2-2 of figure 1.

-3-

DETAILED DESCRIPTION

It has now been found that an improved lyophilization process is obtained when the lyophilization is carried out on shell frozen material. By means of shell freezing, the material to be lyophilized is distributed in a substantially uniform thickness around the sidewall of the vessel or vial in which it is contained leaving a hollow central core devoid of material. As a result of the shell freezing technique, the lyophilization is effected in shorter time and in a more economical fashion, and with reduced consumption of energy per unit volume of material lyophilized.

It is to be emphasized that the present invention is particularly adapted for use on continuous, as opposed to batch, vial filling systems, and can be easily integrated into modern vaccine or pharmaceutical filling lines.

Shell freezing is effected by controlling the relationship between size of the lyophilization vessel and the volume of liquid therein, so that no liquid escapes while the vessel is on its side, and rolling the vessel under rapid freezing conditions. When the material freezes it adheres in a substantially uniform thickness to the inner wall of its container leaving a hollow core. The effect is to greatly increase the ratio of surface area to volume of the material to be lyophilized with the result that lyophilization time is shortened and lyophilization expense is reduced.

By rapid freezing conditions is meant the ability to freeze the contents of the lyophilization vial in a few seconds, typically about 10 seconds or less. This is effected by rotating the vial in a

-4-

liquid bath maintained at a temperature below the eutectic point of the material being frozen. Generally, this temperature is below about -35°C. The vial may be rotated at from about 75 to about 350 rpm when contacting the liquid bath. In general, at lower bath temperatures a faster rate of rotation may be employed, and conversely, at higher bath temperatures a lower rate of rotation may be employed. The distance that the vial travels in the bath also affects the freezing. A shorter distance generally requires a lower bath temperature and a higher rate of rotation, or both, while a longer distance permits a higher bath temperature or a lower rate of rotation, or both. The desired residency time the vial in the bath, typically from about 5 to about 10 seconds, taken with the speed of the filling line, determines the length of the bath.

When rotating a lyophilization vial containing a liquid under rapid freezing conditions, the liquid freezes around the inner sidewall of the vessel. It is important that the frozen product be uniformly and evenly distributed in order to achieve the shortest lyophilization cycle. The standard lyophilization cycle for conventional plug frozen material is in the order of 40 hours. With shell freezing according to the present invention, the lyophilization time is reduced to about 10 hours or less. This is because the path for water out of the product is much shorter in the shell frozen material and because a larger surface area, relative to total volume of the material, is presented for its sublimation.

By shortening the lyophilization time for each lyophilization cycle, not only are costs per unit reduced but, equally as important, the effective capacity of the lyophilization equipment is increased. This is an important consideration due to the high cost of lyophilization equipment.

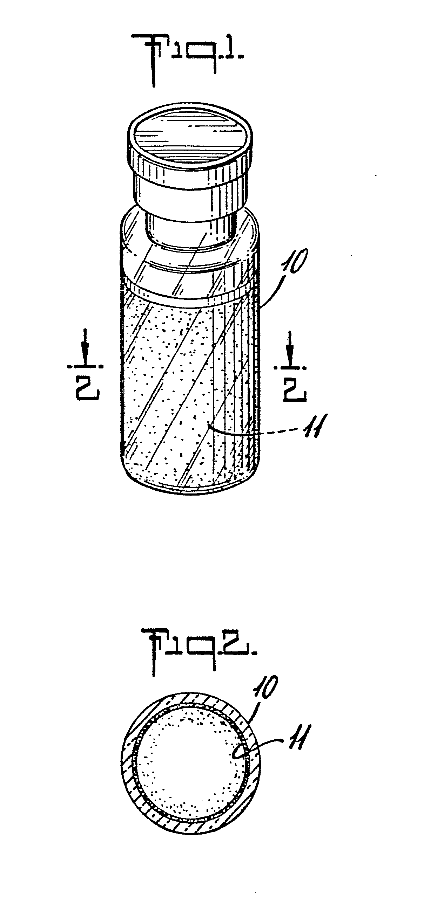

Figure 1 shows a lyophilization vial 10 containing a layer 11 of shell frozen lyophilized vaccine on the inner wall of the vial. The shell frozen vaccine prior to lyophilization is not shown, as it is deemed to be obvious from figure 1 and would differ only in that layer 11 would be thicker and would be a frozen liquid rather than a lyophilized liquid.

-6-

The following example illustrates the present invention without, however, limiting the same thereto.

EXAMPLE

Single dose lyophilization vials 10 having a total volume of 4 ml, are filled with 0.7 ml of mumps vaccine in a standard continuous filling apparatus. As the vials come out of the filling apparatus, they are passed to a Vial Inspection System, Model VIS 100, manufactured by Production Equipment Inc. of Rochelle Park, New Jersey, which has been modified to turn the vials onto a horizontal roller conveyor rather than merely tilting them at a 45° angle. The conveyor rolls the vials at a rate of about 200 RPM. The volume of each vial is such that the liquid volume remains below the neck of the vial when the vial is on its side. While on the roller conveyor, the vials contact the upper region of an ethanol bath maintained at -70°C. The bath has a length of about 100 cm and each vial spends approximately 5 seconds total time rotating in the alcohol bath as it is moving toward the loading machine. The spinning of the vials in the -70°C bath causes the liquid contained within them to freeze in a thin uniform layer 11 over the inner surface of the vial. This is known as shell freezing.

The product at this point is frozen in a uniform and evenly distributed fashion over the inner wall of the vial. After leaving the alcohol bath the vials are returned to the upright position by the roller belt. Stoppers are then inserted and the vials are passed to a freezer where they are maintained in the frozen state while loaded onto trays.

The vials are held in the frozen state until lyophilization. Lyophilization is effected by placing the vials in a lyophilization chamber having a temperature of about -38°C and reducing the pressure to about 0.04 mbar. After 10 minutes the pressure is increased to about 0.133 mbar by injection of air. This pressure is maintained for 4 hours and then reduced to about 0.04 mbar for an additional 2 hours at which time the lyophilization is completed. The total lyophilization time is 6 hours and 10 minutes.

-8-

WHAT IS CLAIMED IS:

1. A vessel containing lyophilized material wherein the material is disposed in a substantially uniform thickness around the inner wall of the vessel leaving a hollow core substantially devoid of lyophilized material.

2. A vessel containing a shell frozen liquid wherein the liquid is disposed in a substantially uniform thickness around the inner wall of the vessel leaving a hollow core substantially devoid of frozen material.

3. A method of preparing a shell frozen vessel according to claim 2 comprising subjecting a vessel containing a liquid to rapid freezing conditions effective to freeze the liquid while rotating the vessel.

4. A method of preparing a vessel containing lyophilized material according to claim 1 comprising shell freezing a liquid contained in the vessel and lyophilizing the shell frozen liquid.

0048194

Fig.1.

10

2
2

11

Fig2.

10

11